# EUROPEAN PATENT SPECIFICATION

(11) **EP 2 920 300 B1**
(45) Date of publication and mention of the grant of the patent: **12.09.2018**
(21) Application number: 13795394.9
(22) Date of filing: 12.11.2013
(51) Int. Cl.: C12N 5/0781, G01N 33/569, C07K 16/10

(54) **ENRICHMENT OF ANTIGEN-SPECIFIC PLASMABLASTS**
ANREICHERUNG VON ANTIGENSPEZIFISCHEN PLASMABLASTEN
ENRICHISSEMENT DE PLASMOBLASTES SPÉCIFIQUES D'ANTIGÈNES

(30) Priority: 13.11.2012 US 201261725764 P
(43) Date of publication of application: 23.09.2015
(73) Proprietor: F. Hoffmann-La Roche AG, 4070 Basel (CH)
(72) Inventor: ZHANG, Juan, South San Francisco, California 94080 (US); BALAZS, Mercedesz, Seattle, Washington 98199 (US); CHAI, Ning, South San Francisco, California 94080 (US); CHIU, Henry, South San Francisco, California 94080 (US); LEE, Wyne P., South San Francisco, California 94080 (US); LIN, Zhonghua, South San Francisco, California 94080 (US); LUPARDUS, Patrick J., South San Francisco, California 94080 (US); SWEM, Lee R., South San Francisco, California 94080 (US)
(74) Representative: Brodbeck, Michel
(86) International application number: PCT/US2013/069588
(87) International publication number: WO 2014/078280

(56) References cited:
- WO-A1-93/05796
- WO-A1-96/40252
- WO-A1-97/47654
- WO-A1-99/60846
- WO-A1-2011/008093
- WO-A1-2013/030799
- WO-A2-2006/073941
- WO-A2-2010/059876
- US-A- 5 663 481
- DEPRAETERE S ET AL: "Human B cell growth and differentiation in the spleen of immunodeficient mice", THE JOURNAL OF IMMUNOLOGY, THE AMERICAN ASSOCIATION OF IMMUNOLOGISTS, US, vol. 166, no. 5, 1 January 2001 (2001-01-01), pages 2929-2936, XP002993493, ISSN: 0022-1767 cited in the application
- BRAMS ET AL: "Antigen-specific IgG responses from naive human splenocytes: in vitro priming followed by antigen boost in the SCID mouse.", THE JOURNAL OF IMMUNOLOGY, vol. 160, no. 5, 1 March 1998 (1998-03-01) , pages 2051-2058, XP055103900, ISSN: 0022-1767 cited in the application
- BOCHER ET AL: "Antigen-specific B and T cells in human/mouse radiation chimera following immunization in vivo", IMMUNOLOGY, vol. 96, no. 4, 1 April 1999 (1999-04-01), pages 634-641, XP055103754, ISSN: 0019-2805, DOI: 10.1046/j.1365-2567.1999.00704.x
- DAVIDE CORTI ET AL: "A Neutralizing Antibody Selected from Plasma Cells That Binds to Group 1 and Group 2 Influenza A Hemagglutinins", SCIENCE, AMERICAN ASSOCIATION FOR THE ADVANCEMENT OF SCIENCE; THE INSTITUTION OF ELECTRICAL ENGINEERS, STEVENAGE, GB, vol. 333, no. 6044, 12 August 2011 (2011-08-12), pages 850-856, XP002689150, ISSN: 0036-8075, DOI: 10.1126/SCIENCE.1205669 [retrieved on 2011-07-28] cited in the application
- KYU S Y ET AL: "Frequencies of human influenza-specific antibody secreting cells or plasmablasts post vaccination from fresh and frozen peripheral blood mononuclear cells", JOURNAL OF IMMUNOLOGICAL METHODS, ELSEVIER SCIENCE PUBLISHERS B.V.,AMSTERDAM, NL, vol. 340, no. 1, 1 January 2009 (2009-01-01), pages 42-47, XP025991298, ISSN: 0022-1759, DOI: 10.1016/J.JIM.2008.09.025 [retrieved on 2008-11-06]
- GERALD NAKAMURA ET AL: "An In Vivo Human-Plasmablast Enrichment Technique Allows Rapid Identification of Therapeutic Influenza A Antibodies", CELL HOST & MICROBE, vol. 14, no. 1, 1 July 2013 (2013-07-01), pages 93-103, XP055103733, ISSN: 1931-3128, DOI: 10.1016/j.chom.2013.06.004
- JONATHAN W. YEWDELL ET AL: "Greasing the SCIDs for Universal Flu Antibodies", CELL HOST & MICROBE, vol. 14, no. 1, 1 July 2013 (2013-07-01), pages 7-8, XP055104145, ISSN: 1931-3128, DOI: 10.1016/j.chom.2013.07.002

## Description

### RELATED APPLICATIONS

This application claims the benefit of U.S. Provisional Application No. 61/725,764, filed on 13 November 2012.

### SEQUENCE LISTING

The instant application contains a Sequence Listing which has been submitted electronically in ASCII format and is hereby incorporated by reference in its entirety. Said ASCII copy, created on October 22, 2013, is named P5500RI-WO_SL.txt and is 15,453 bytes in size.

### FIELD OF THE INVENTION

The present invention relates to methods for enrichment of antigen-specific plasmablasts. The methods are useful for the identification of antibodies, including rare antibodies which recognize more than one antigen.

### BACKGROUND

Technological advances which enable the cloning of human immunoglobulin genes have proven effective for the discovery of rare functional human monoclonal antibodies with therapeutic potential. However, many methods used to discover such rare human antibodies are often laborious, involving *in vitro* cultivation and sorting of hundreds of thousands of human plasma cells or the generation of large phage-display libraries from human plasma cells to identify only a few potential antibody candidates.

Various methods have been developed for increasing the efficiency of identifying rare functional human monoclonal antibodies. Brams *et al.* reported antigen-specific IgG responses by combining *in vitro* priming of naive human splenocytes with antigen followed by intraperitoneal or intravenous transfer of the primed splenocytes into immunocompromised mice followed by a subsequent *in vivo* boosting of the donor mice with additional antigen. (See Brams et al., (1998) J Immunology 160:2051-2058; International Application Publication No. WO 1996/40252.) A requirement for *in vitro* spleen cell cultivation and both *in vitro* and *in vivo* antigen challenge was noted in order to achieve enhancement of an antigen-specific response. Others reported antigen-selective human B cell expansion following engraftment of human peripheral blood leukocytes (PBLs) together with antigen directly into the spleens of immunocompromised mice. (See Depraetere et al., (2001) J Immunology 166:2929-2936; International Application Publication NO. WO 1999/60846.)

Despite these advances in antibody identification techniques, a need exists for methods for enrichment of antigen-specific plasmablasts useful for the identification of rare monoclonal antibodies, including monoclonal antibodies which recognize more than one antigen. The present inventors have developed an *in vivo* plasmablast enrichment technique effective for identifying antigen-specific plasmablasts. Described herein are *in vivo* antigen-driven plasmablast enrichment techniques which, when used in combination with antigen-specific cell sorting, allow for efficient and high frequency identification of rare and functional monoclonal antibodies without the need for plasma cell propagation or generation of phage-display libraries. The methods provided by the present invention are particularly useful for identifying rare antibodies. The methods provided by the present invention are also particularly useful for identifying an antibody (e.g., a monoclonal antibody) having specificity to more than one antigen.

### SUMMARY OF THE INVENTION

The present invention provides, in part, methods for enriching antigen-specific plasmablasts.

In some aspects, the present invention provides methods for enriching antigen-specific plasmablasts, the method comprising obtaining peripheral blood mononuclear cells (PBMCs) from a donor, contacting *in vitro* the PBMCs with at least one antigen, transferring the PBMCs into the spleen of an immunocompromised non-human animal, and isolating the plasmablasts from the spleen of the immunocompromised animal, thereby enriching antigen-specific plasmablasts. In some embodiments, the additional step of washing the PBMCs to remove excess or unbound antigen after contacting *in vitro* the PBMCs with the antigen and prior to transferring the PBMCs into the spleen of the immunocompromised animal is performed. In some embodiments, the additional step of antigen-specific single-cell sorting of the plasmablasts is performed. In other embodiments, the additional step of immunoglobulin cloning from the isolated plasmablasts is performed.

In some aspects, the present invention provides methods for enriching antigen-specific plasmablasts, the method comprising obtaining peripheral blood mononuclear cells (PBMCs) from a donor, contacting *in vitro* the PBMCs with at least one antigen, washing the PBMCs to remove excess or unbound antigen, transferring the PBMCs into the spleen of an immunocompromised non-human animal, and isolating the plasmablasts from the spleen of the immunocompromised animal, thereby enriching antigen-specific plasmablasts. In some embodiments, the additional step of antigen-specific single-cell sorting of the plasmablasts is performed. In other embodiments, the additional step of immunoglobulin cloning from the isolated plasmablasts is performed.

In some aspects, the PBMCs are obtained from a donor previously exposed to an antigen. Accordingly, in some aspects, the present invention provides methods for enriching antigen-specific plasmablasts, the method comprising obtaining peripheral blood mononuclear cells (PBMCs) from a donor previously exposed to an antigen, contacting *in vitro* the PBMCs with the antigen or a fragment or portion thereof, transferring the PBMCs into the spleen of an immunocompromised non-human animal, and isolating the plasmablasts from the spleen of the immunocompromised animal, thereby enriching antigen-specific plasmablasts. In some embodiments, the additional step of washing the PBMCs to remove excess or unbound antigen after contacting *in vitro* the PBMCs with the antigen and prior to transferring the PBMCs into the spleen of the immunocompromised animal is performed. In some embodiments, the additional step of antigen-specific single-cell sorting of the plasmablasts is performed. In other embodiments, the additional step of immunoglobulin cloning from the isolated plasmablasts is performed.

In some aspects, the present invention provides methods for identifying or isolating an antibody, the method comprising obtaining peripheral blood mononuclear cells (PBMCs) from a donor, contacting *in vitro* the PBMCs with at least one antigen, transferring the PBMCs into the spleen of an immunocompromised non-human animal, isolating the plasmablasts from the spleen of the immunocompromised animal, and sorting the isolated antigen-specific plasmablasts, thereby identifying or isolating an antibody. In some embodiments, the additional step of washing the PBMCs to remove excess or unbound antigen after contacting *in vitro* the PBMCs with the antigen and prior to transferring the PBMCs into the spleen of the immunocompromised animal is performed. In some embodiments, the additional step of immunoglobulin cloning from the isolated plasmablasts is performed.

In some aspects of the present methods, the antigen used in the step of contacting *in vitro* the PBMCs with antigen is the same antigen (or a fragment or portion thereof) as the antigen to which the donor was previously exposed. In other embodiments, the antigen used in the step of contacting *in vitro* the PBMCs with antigen is a different antigen from the antigen to which the donor was previously exposed (*e.g.,* the antigen is a variant, a subtype, an isotype, or a homolog of the antigen to which the donor was previously exposed).

In some aspects, the present invention provides methods for obtaining a plasmablast population enriched for plasmablasts having specificity to an antigen, the method comprising obtaining peripheral blood mononuclear cells (PBMCs) from a donor, contacting *in vitro* the PBMCs with the antigen or a fragment or portion thereof, transferring the PBMCs into the spleen of an immunocompromised non-human animal, and isolating the plasmablasts from the spleen of the immunocompromised animal, thereby obtaining a plasmablast population enriched for plasmablasts having specificity to an antigen. In some embodiments, the additional step of washing the PBMCs to remove excess or unbound antigen after contacting *in vitro* the PBMCs with the antigen and prior to transferring the PBMCs into the spleen of the immunocompromised animal is performed. In some embodiments, the enriched plasmablast population contains at least one antigen-specific plasmablast having specificity to the antigen. In some embodiments, the enriched plasmablast population contains at least one antigen-specific antibody-producing plasmablast having specificity to the antigen. In some embodiments, the additional step of antigen-specific single-cell sorting of the plasmablasts is performed. In other embodiments, the additional step of immunoglobulin cloning from the isolated plasmablasts is performed.

In other aspects, the present invention provides methods for obtaining a plasmablast population enriched for plasmablasts having specificity to an antigen, the method comprising obtaining peripheral blood mononuclear cells (PBMCs) from a donor previously exposed to the antigen, contacting *in vitro* the PBMCs with the antigen or a fragment or portion thereof, transferring the PBMCs into the spleen of an immunocompromised non-human animal, and isolating the plasmablasts from the spleen of the immunocompromised animal, thereby obtaining a plasmablast population enriched for plasmablasts having specificity to an antigen. In some embodiments, the additional step of washing the PBMCs to remove excess or unbound antigen after contacting *in vitro* the PBMCs with the antigen and prior to transferring the PBMCs into the spleen of the immunocompromised animal is performed. In some embodiments, the enriched plasmablast population contains at least one antigen-specific plasmablast having specificity to the antigen. In some embodiments, the enriched plasmablast population contains at least one antigen-specific antibody-producing plasmablast having specificity to the antigen. In some embodiments, the additional step of antigen-specific single-cell sorting of the plasmablasts is performed. In other embodiments, the additional step of immunoglobulin cloning from the isolated plasmablasts is performed.

In other aspects, the present invention provides methods for a identifying a plasmablast capable of producing an antibody having specificity to more than one antigen, the method comprising obtaining peripheral blood mononuclear cells (PBMCs) from a donor, contacting *in vitro* the PBMCs with two or more different antigens to obtain a PBMC/antigen pre-mix, transferring the PBMCs into the spleen of an immunocompromised non-human animal, isolating the plasmablasts from the spleen of the immunocompromised animal, and antigen-specific cell sorting of the isolated plasmablasts, thereby identifying a plasmablast capable of producing an antibody having specificity to an antigen. In some embodiments, the method further comprises washing the PBMCs to remove excess or unbound antigen from the PBMC/antigen pre-mix prior to transferring the PBMCs into the spleen of the immunocompromised. In other embodiments, the method further comprises cloning of the immunoglobulin from the isolated plasmablast.

In some aspects, the PBMCs are obtained from a mammal. In some embodiments, the PBMCs are obtained from a mouse, a rat, a rabbit, or a goat. In other embodiments, the PBMCs are obtained from a human. In other aspects, the PBMCs are obtained from an animal which is a non-mammal.

### BRIEF DESCRIPTION OF THE FIGURES

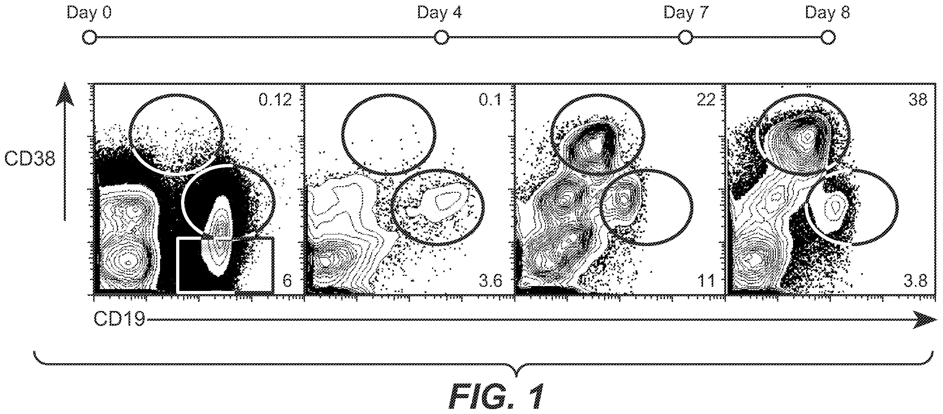

Figure 1 sets forth data showing plasmablast differentiation in SCID mice *in vivo.* Human peripheral blood mononuclear cells (PBMCs) prior to transplant (indicated as day 0) or splenocytes obtained from SCID mice on day 4, 7, and 8 (following intrasplenic injection of human PBMCs) were stained with anti-CD 19 and anti-CD38 to distinguish B lineage cells. Upper circles, lower circles, and rectangles represent plasmablasts, activated germinal center-like cells, or naive B cells, respectively. Numbers indicate frequencies of each subset as percent.

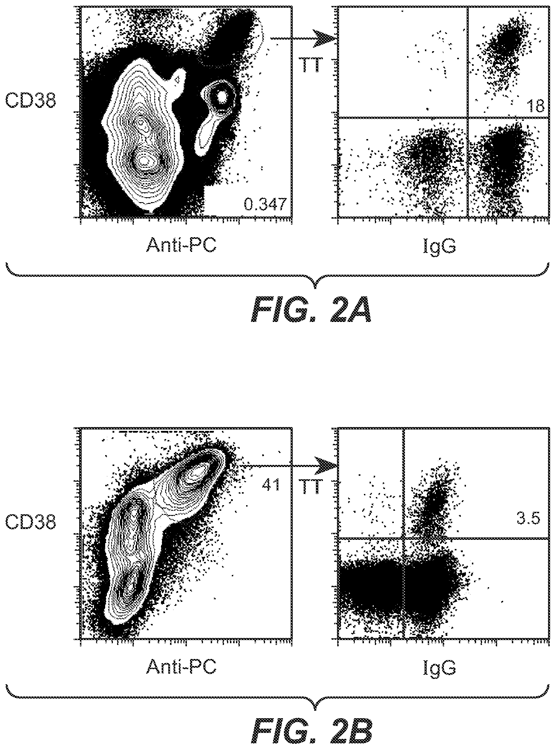

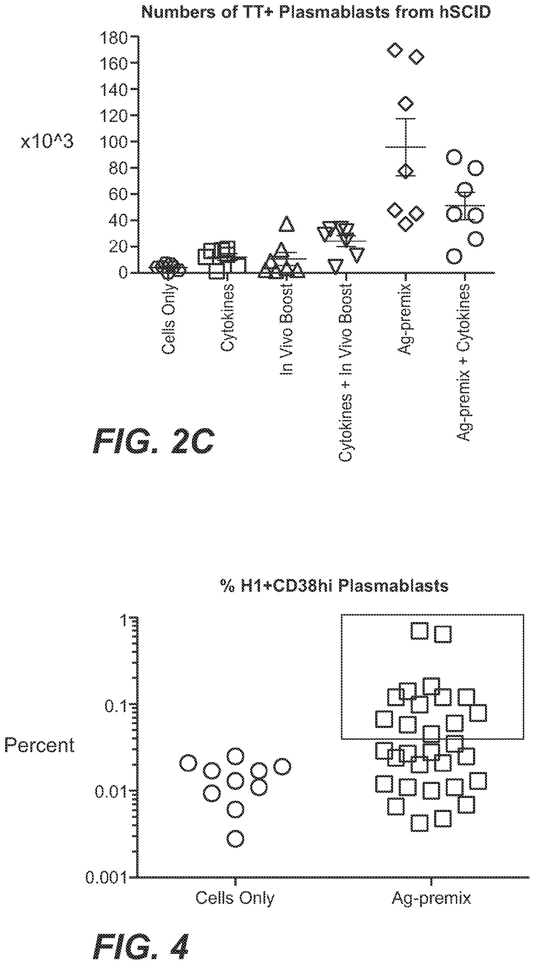

Figure 2A shows a representative profile of human donor PBMCs (tetanus toxoid reactive) at day 7 post tetanus vaccination. Figure 2B shows a representative profile of spleen cells (tetanus toxoid reactive) obtained from SCID mice at day 8 after transplantation of human PBMCs obtained from tetanus vaccinated donors. Numbers indicate frequencies of each subset as percent. Figure 2C shows the number of tetanus toxoid reactive cells per spleen obtained from individual SCID mice under each stimulation condition. *In vivo* boost contained 100 µg tetanus toxoid injected IP to SCID mice. Cytokine cocktail contained a mixture of human B cell activating factor (BAFF, 50µg) and IL-2, IL-6, and IL-21 (50 ng each) injected intra peritoneal (i.p.) to SCID mice.

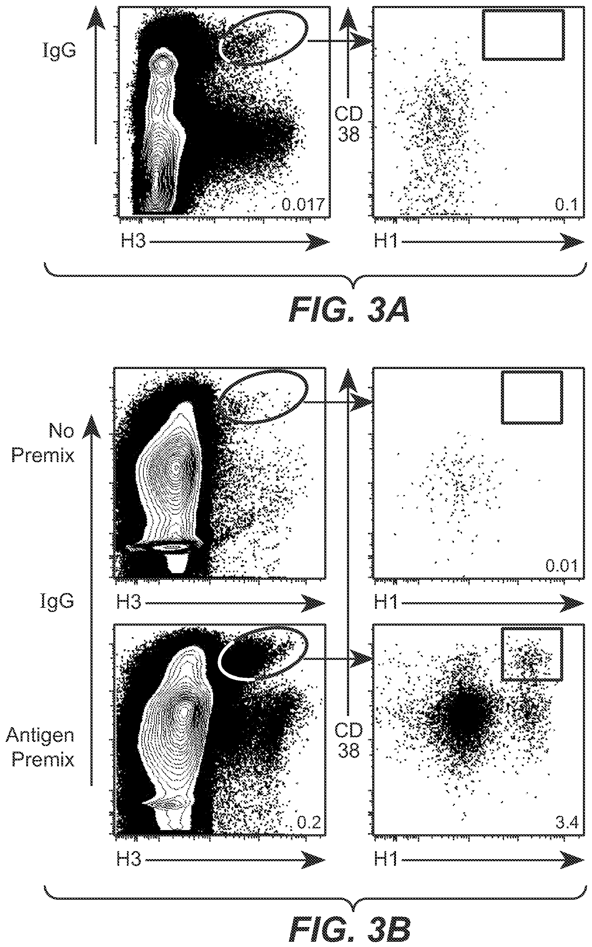

Figures 3A and 3B sets forth data showing FACS analysis of anti-hemagglutinin-positive plasmablasts (H3 hemagglutinin and H1 hemagglutinin) from day 7 post-vaccinated PBMCs prior to SCID mouse enrichment (Figure 3A) and day 8 post-transplant after SCID mouse enrichment with and without antigen premix (Figure 3B) in the upper and lower panels, respectively.
Figure 4 sets forth data showing analysis of splenocytes obtained from day 8 post-intrasplenic transplant of PBMCs from individual SCID/beige mice with no PBMC/antigen premix (circles) and with PBMC/antigen premix (squares), as percent hemagglutinin (H1)⁺/CD38^{high} plasmablasts. The rectangle indicates mice that presented H1⁺ plasmablasts.

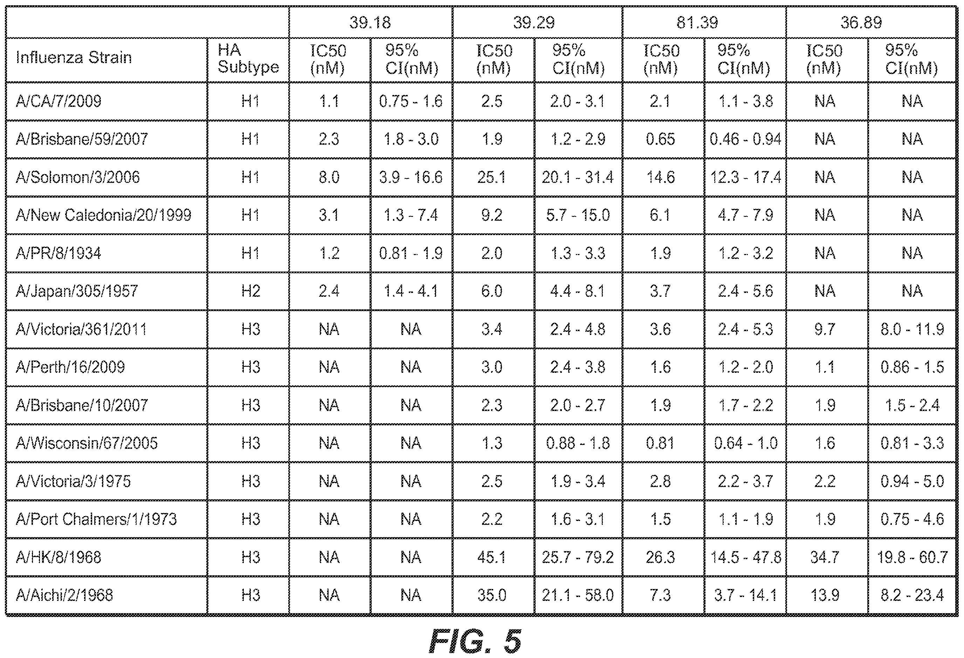

Figure 5 sets for data showing in vitro neutralization of various influenza A Group1 and Group2 virus strains by anti-hemagglutinin antibodies obtained using methods of the present invention.

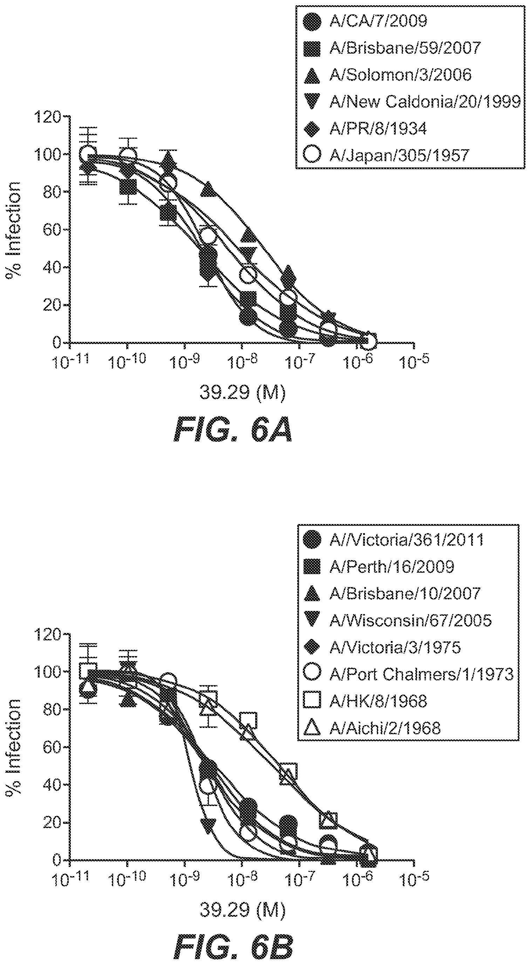

Figures 6A and 6B set forth data showing *in vitro* neutralization of various influenza A Group1 (Figure 6A) and Group2 (Figure 6B) virus strains by monoclonal antibody 39.29.

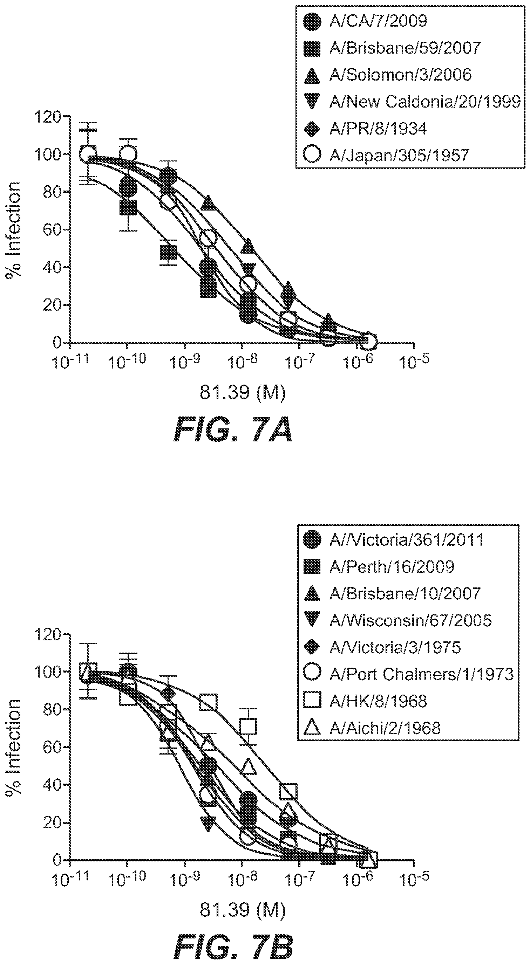

Figures 7A and 7B set forth data showing *in vitro* neutralization of various influenza A Group 1 (Figure 7A) and Group2 (Figure 7B) virus strains by monoclonal antibody 81.39.

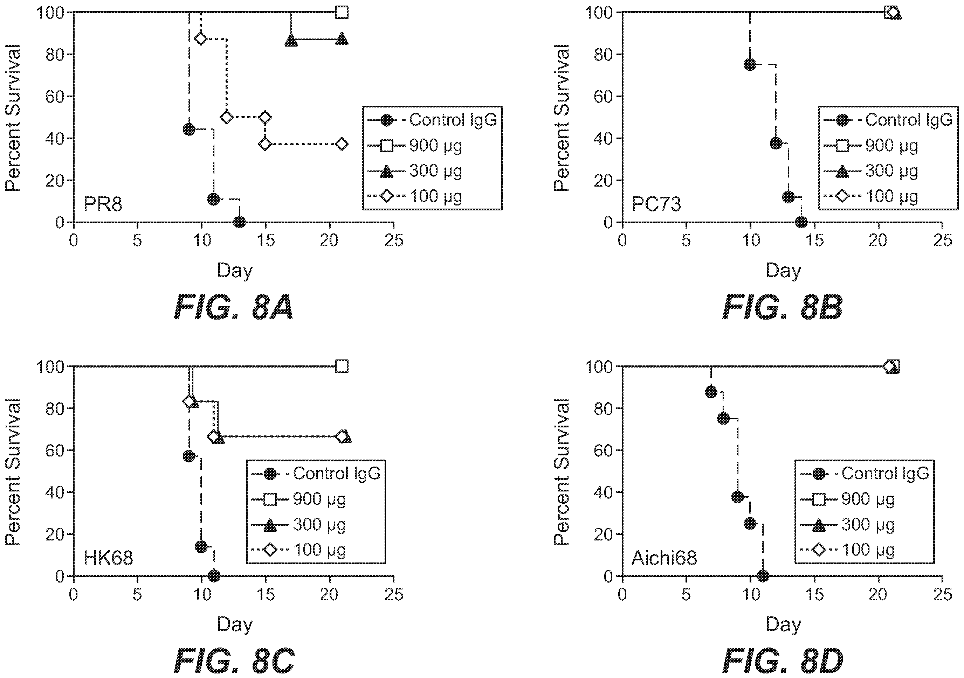

Figures 8A, 8B, 8C, and 8D set forth data showing percent survival of mice infected with various influenza A virus strains (A/PR/8/1934 (PR8), Figure 8A; A/Port Chalmers/1/1973 (PC73), Figure 8B; A/Hong Kong/1/1968 (HK68), Figure 8C); and A/Aichi/2/1968 (Aichi68), Figure 8D) and administered various amounts of monoclonal antibody 39.29.

### DETAILED DESCRIPTION OF EMBODIMENTS OF THE INVENTION

The present invention provides, *inter alia,* methods for antigen-specific enrichment of plasmablasts. The present methods provide for rapid identification of antigen-specific plasmablasts and efficient identification of rare monoclonal antibodies.

### Definitions

The term "plasmablast" refers to a cell which is a precursor to a plasma cell.

The term "splenocytes" refers to any one of the different blood cell types located in the spleen or obtained from splenic tissue.

The term "enriching" or "enrichment" of plasmablasts refers to increasing the frequency of desired cells, typically antigen-specific cells, contained in a mixed cell population. Thus, enriching or an enrichment of a cell population (*e.g.,* antigen-specific plasmablasts) encompasses a cell population having a higher frequency of antigen-specific cells (*e.g.,* antigen-specific plasmablasts) as a result of an enriching or enrichment step.

"Peripheral blood mononuclear cells" (PBMCs) refers to blood cells having a round nucleus, and includes lymphocytes (T cell and B cells), monocytes, macrophages, natural killer cells, plasma cells, and plasmablasts. As used herein, the term "peripheral blood mononuclear cells" is used interchangeably with the term "peripheral blood lymphocytes" (PBLs), which includes lymphocytes (*e.g.,* B cells, T cells, natural killer cells).

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e.g.,* scFv); and multispecific antibodies formed from antibody fragments.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g.,* IgG₁, IgG₂, IgG₃, IgG₄, IgA₁, and IgA₂. The heavy chain constant domains that correspond to the different classes of immunoglobulins are called α, δ, ε, γ, and µ, respectively.

An "effective amount" of an agent, *e.g.,* a pharmaceutical formulation, refers to an amount effective, at dosages and for periods of time necessary, to achieve the desired therapeutic or prophylactic result.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human hyper variable regions (HVRs) and amino acid residues from human framework regions (FRs). In certain embodiments, a humanized antibody will comprise substantially all of at least one, and typically two, variable domains, in which all or substantially all of the HVRs (*e.g.,* CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

An "individual" or "subject" is a mammal. Mammals include, but are not limited to, domesticated animals *(e.g.,* cows, sheep, cats, dogs, and horses), primates *(e.g.,* humans and non-human primates such as monkeys), rabbits, and rodents (*e.g.,* mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (*e.g.,* SDS-PAGE, isoelectric focusing (IEF), capillary electrophoresis) or chromatographic (*e.g.,* ion exchange or reverse phase HPLC). For review of methods for assessment of antibody purity, see, *e.g.,* Flatman et al., J. Chromatogr. B 848:79-87 (2007).

The term "monoclonal antibody" as used herein refers to an antibody obtained from a population of substantially homogeneous antibodies, *i.e.,* the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis. In some embodiments, antibodies of the invention are used to delay development of a disease or to slow the progression of a disease.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### General Methods

The practice of the present invention will employ, unless otherwise indicated, conventional techniques of cell biology, cell culture, molecular biology (including recombinant techniques), microbiology, biochemistry, and immunology, which are known and available to one of skill in the art. Such techniques are described in the literature, such as, Molecular Cloning: A laboratory Manual, third edition (Sambrook et al., 2001) Cold Spring Harbor Press; Oligonucleotide Synthesis (P. Herdewijn, ed., 2004); Animal Cell Culture (R.I. Freshney, ed., 1987); Methods in Enzymology (Academic Press, Inc.); Current Protocols in Molecular Biology (F.M. Ausubel et al., eds., 1987); PCR: The Polymerase Chain Reaction (Mullis et al., eds., 1994); Current Protocols in Immunology (J.E. Coligan et al., eds., 1991); and Short Protocols in Molecular Biology (Wiley and Sons, 1999). Expression of antibody fragments and polypeptides in bacteria are described in, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*)

Unless defined otherwise, all technical and scientific terms used herein have the same meaning as is commonly understood by one of ordinary skill in the art to which the invention belongs.

Provided herein are methods useful for the identification of rare monoclonal antibodies. In particular, the present invention provides an *in vivo* plasmablast enrichment methodology for efficient and effective identification of antigen-specific plasmablasts, from which antibodies, and the nucleic acids encoding such antibodies, can be isolated. It is one particular aspect of the present invention to provide methods for the identification and isolation of rare antibodies using *in vivo* antigen-specific enrichment of plasmablasts, wherein the antibodies are rare functional monoclonal antibodies. It is another particular aspect of the present invention to provide methods for the identification and isolation of antibodies which recognize (*e.g.,* bind to) more than one antigen (*e.g.,* recognize or bind to variants, isotypes, subtypes, or homologs of the same antigen or to the same antigen of different species). The present methods include obtaining (*e.g.,* isolating) peripheral blood mononuclear cells (PBMCs) from a donor, contacting *in vitro* the PBMCs with antigen (herein referred to as PBMC/antigen pre-mix), transferring (by injection, transplantation, or other means) the PBMCs into the spleen of an immunocompromised non-human animal, allowing the injected PBMCs to reside or remain in the spleen of the immunocompromised animal, and isolating the antigen-specific plasmablasts from the spleen. Methods for isolating plasmablasts from spleen are well-known and available to one skilled in the art.

Contacting the PBMCs with antigen is performed *in vitro.* In some embodiments, unbound antigen is not washed from the PBMCs following the PBMC/antigen pre-mix and prior to transferring the PBMCs into the spleen of an immunocompromised non-human animal. In other embodiments, an additional step of washing the PBMCs to remove unbound antigen is performed following PBMC/antigen pre-mix and prior to transferring the PBMCs into the spleen of an immunocompromised non-human animal. In some embodiments, the additional step of antigen-specific single-cell sorting is performed following the isolating of the antigen-specific plasmablasts from the spleen. In other embodiments, the additional step of immunoglobulin cloning from the isolated cells is performed following the isolating of the antigen-specific plasmablasts from the spleen or following the single-cell sorting. Optimization of the methods for *in vivo* plasmablast enrichment was performed using tetanus toxoid antigen. In these studies, the inventors showed that increased numbers of tetanus toxoid-specific plasmablasts were obtained using the methods described herein. (See Examples 1 and 2.) The methods were further applied to the enrichment and identification of rare plasmablasts specific for hemagglutinin from influenza A virus. Use of the plasmablast enrichment methods of the present invention resulted in the identification of human monoclonal antibodies effective at binding and neutralizing various influenza A virus isolates, including human monoclonal antibodies effective at binding both Group 1 and Group2 hemagglutinin, and/or effective at neutralizing both Group 1 and Group2 influenza A virus isolates. (See Examples 3-7.)

The present methods provide advantages over previous methods for identifying monoclonal antibodies, including, but not limited to, efficient and enhanced antigen-specific plasmablast enrichment, increased antigen-specific response, identification of antibodies having high affinity and function, and identification of antibodies which recognize more than one antigen.

The methods of the present invention provide additional advantages over prior art methods by increasing the efficiency of identifying rare functional monoclonal antibodies. For example, the present methods are effective without *in vivo* immunization of the immunocompromised animal (*e.g.,* mice) with the antigen or antigens of interest at the time of or subsequent to transfer of the PBMCs into the spleen of the immunocompromised animal. Additionally, the present methods are effective without the immunocompromised animal being boosted with the antigen or antigens of interest at the time of or subsequent to transfer of the PBMCs into the spleen of the immunocompromised animal. Furthermore, the methods of the present invention are not associated with removal or killing of the immunocompromised animal's natural killer (NK) cells, such as by pretreatment of the animal with anti-IL-2-Rb antibodies prior to transfer of the PBMCs into the spleens of the immunocompromised animal, in order to enrich for antigen-specific plasmablasts.

In the context of identification of anti-hemagglutinin antibodies (for use in treating or preventing influenza virus infection), a previous report described direct cloning of hemagglutinin binding antibodies from approximately 104,000 *in vitro* cultured human plasma cells to discover a single antibody that could bind and neutralize all Group1 and Group2 influenza A virus isolates. (See Corti et al., (2011) Science 333:850-856.) The plasmablast enrichment methods described herein, however, resulted in the identification of twenty anti-hemagglutinin antibodies which recognized various hemagglutinin subtypes, of which four anti-hemagglutinin antibodies were effective at neutralizing various Group1 and Group2 influenza A virus isolates by screening only 840 cloned human monoclonal antibodies.

The antigen-specific plasmablast enrichment methods of the present invention are useful to identify plasmablasts and their corresponding antibodies to any desired antigen, including, for example, tetanus toxoid, rubella, measles, mumps, varicella zoster, toxoplasmosis, influenza virus, HIV, hepatitis A, hepatitis B, hepatitis C, papillomavirus, yeast, parasites (*e.g.,* malaria), bacteria, CMV, rous sarcoma virus, etc.

PBMCs for use in the present methods can be obtained from any animal (the donor) from which an antigen-specific antibody is desired. Such animals include, for example, mice, rats, rabbits, chickens, goats, humans, etc. In some embodiments, PBMCs are obtained from a mammal, such as, for example, a human. In other embodiments, PBMCs are obtained from a human previously immunized with or exposed to at least one antigen, infectious agent (*e.g.,* a bacterial or viral agent), or parts thereof, wherein the antigen is sufficient to illicit an immune response to the antigen. Alternatively, PBMCs can be obtained from a non-human animal, in particular a non-human mammal. Accordingly, in some embodiments, PBMCs are obtained from a non-human animal previously immunized with or exposed to at least one antigen, infectious agent, or parts thereof. The antigen can be any self- or non-self-antigen. In particular embodiments, the antigen is capable of inducing an immune response in the donor which leads to the presence of antigen-specific plasmablasts, plasma cells, or B cells in the hematopoietic and lymphomoncytic tissues.

The present methods can be applied to any immunocompromised non-human animal, including mice or other non-human animal species. Immunocompromised animals typically will have limited or will lack the ability to evoke an effective immune response against an implanted xenograft or allograft blood leukocytes/lymphcytes (*e.g.,* PBMCs, PBLs) or splenocytes. In certain aspects, the immunocompromised animals have no functional T cells or B cells that may otherwise reject the implanted cells. Examples of immunocompromised non-human animals for use in the present methods include, for example, SCID/beige mice, NOD-SCID mice, nude mice, etc.

In some aspects, the antigen for use in the PBMC/antigen pre-mix is a purified or substantially purified antigen. The antigen can be an antigen produced by recombinant means (*i.e.,* a recombinant antigen) or naturally produced. The antigen can be a full-length protein or polypeptide, or can comprise a portion or fragment of a protein or polypeptide, such as, for example, a proteolytic fragment. The antigen can also be a synthetic protein or polypeptide produced by synthetic means. Additionally, the antigen for use in the PBMC/antigen pre-mix can include one antigen or more than one antigen (*e.g.,* a mixture of more than one antigen). For example, the antigen may be a mixture of more than one antigen, such as, for example, more than one hemagglutinin subtype from different influenza A virus isolates (*e.g.,* Group1 and Group2 hemagglutinin subtypes).

The amount of antigen used for the PBMC/antigen pre-mix according to the present methods may vary. The present invention provides exemplary data showing that about 0.1 to 2 µg of antigen for each one million B cells in the PBMC/antigen pre-mix resulted in effective and robust enrichment of antigen-specific plasmablasts. The present methods, however, are not limited to the amount of antigen used in the PBMC/antigen pre-mix. Generally, the amount of antigen used for the PBMC/antigen pre-mix will be about 0.001 µg, 0.005 µg, 0.01 µg, 0.05 µg, 0.1 µg, 0.2 µg, 0.3 µg, 0.4 µg, 0.5 µg, 0.75 µg, 1.0 µg, 2 µg, 5 µg, or 10 µg per 1 million B cells; or in a range of about 0.001 µg to 10 µg per 1 million B cells. It is well within the skill of one in the art to determine (by routine experimentation) the amount of antigen used for the PBMC/antigen pre-mix for enrichment of antigen-specific plasmablasts.

The length of time for the PBMC/antigen pre-mix according to the present methods may vary. The present invention provides exemplary data showing that a 30 minute PBMC/antigen premix resulted in effective and robust enrichment of antigen-specific plasmablasts. Generally, the length of time for PBMC/antigen pre-mix will be about 10 minutes to about 180 minutes, such as, for example, about 10 minutes, about 15 minutes, about 20 minutes, about 30 minutes, about 45 minutes, about 60 minutes, about 75 minutes, about 90 minutes, about 120 minutes, about 150 minutes, or about 180 minutes. It is well within the skill of one in the art to determine (by routine experimentation) the length of time for the PBMC/antigen pre-mix for enrichment of antigen-specific plasmablasts.

The temperature at which the PBMC/antigen pre-mix occurs according to the present methods may vary. The present invention shows by way of example that performing the PBMC/antigen pre-mix at 37°C (for 30 minutes) resulted in effective and robust enrichment of antigen-specific plasmablasts. It is well within the skill of one in the art to determine (by routine experimentation) the temperature at which the PBMC/antigen pre-mix occurs. Generally, lower temperatures may require longer PBMC/antigen pre-mix times. For example, performing the PBMC/antigen pre-mix at 30°C rather than at 37°C may require an antigen premix time of longer than 30 minutes, such as, for example, about 45 minutes, about 60 minutes, about 90 minutes, etc.

In some aspects, the PBMC/antigen pre-mix can be performed in the absence or presence of an adjuvant, e.g., Complete Freund's Adjuvant, Alum, Saponin, etc. Other known adjuvants may also be used in the PBMC/antigen pre-mix.

Any number of cells can be mixed or contacted *in vitro* with antigen in the PBMC/antigen pre-mix step. The present methods are not limited to the number of cells contacted *in vitro* with antigen in the PBMC/antigen pre-mix. It is well within the skill of one in the art to determine (by routine experimentation) the number of cells used for the PBMC/antigen pre-mix for enrichment of antigen-specific plasmablasts.

The number of cells (*e.g.,* PBMCs) implanted into the spleen of an immunocompromised non-human animal according to the present methods may also vary. The present invention provides exemplary data showing that 50x10⁶ PBMCs implanted into the spleens of immunocompromised mice resulted in the successful identification of rare antigen-specific plasmablasts. The present methods, however, are not limited to the number of cells implanted into the spleen of an immunocompromised non-human animal. It is well within the skill of one in the art to determine (by routine experimentation) the number of cells to implant into the spleen of an immunocompromised non-human animal. Generally, the number of cells will be about 1x10⁵, about 1x10⁶, about 5x10⁶, about 1x10⁷, about 5x10⁷, about 1x10⁸, about 5x10⁸, about 1x10⁹, about 5x10⁹, or about 1x10¹⁰.

Following intra-splenic implantation, one can allow the implanted PBMCs to remain in the spleen for various amounts of time before being isolated. The present invention provides exemplary data showing that allowing the implanted PBMCs to remain within the spleen of the immunocompromised mice for 7 or 8 days resulted in effective and robust enrichment of antigen-specific plasmablasts to tetanus toxoid and hemagglutinin. Generally, the length of time the implanted PBMCs remain within the spleen before being isolated will be between about 4 days to about 14 days, about 7 days to about 10 days, about 7 days, or about 8 days. However, the implanted PBMCs can remain within the spleen following intra-splenic injection for about 1 day, about 2 days, about 3 days, about 4 days, about 5 days, about 6 days, about 7 days, about 8 days, about 9 days, about 10 days, about 11 days, about 12 days, about 13 days, about 14 days, or more than 14 days. Beyond 14 days, it is expected or anticipated that the implanted PBMCs may initiate an attack on the mouse natural killer (NK) cells. Intra-splenic transplantation of fewer cells may allow for longer times for the implanted PBMCs to remain in the spleen prior to their isolation. It is well within the skill of one in the art to determine (by routine experimentation) the length of time for the PBMCs to remain in the spleen prior to isolation for enrichment of antigen-specific plasmablasts.

In some embodiments, the PBMCs are obtained from one individual donor. In other embodiments, the PBMCs are obtained from more than one individual donor. PBMCs obtained from more than one donor can be pooled or mixed together prior to PBMC/antigen pre-mix or prior to intra-splenic injection following PBMC/antigen pre-mix.

The present methods are not limited to the use of PBMCs or PBLs for enrichment of antigen-specific plasmablasts obtained from a donor. Other donor cell sources for plasmablasts and B cells can be used with the present methods, such as, for example, spleen cells, bone marrow cells, tonsillar cells, cells obtained from lymph glands, stem cells, etc. For example, in certain aspects, splenocytes (*i.e.,* spleen cells) may be obtained from a donor for use in enrichment of antigen-specific plasmablasts according to the present invention. Splenocytes may be isolated from a mouse immunized with one or more antigens, mixed, incubated, or contacted with antigen (antigen pre-mix) according to the methods described herein, and subsequently injected into the spleen of an immunocompromised non-human animal. Prior to antigen pre-mix, the splenocytes may be enriched for mononuclear cells (*e.g.,* plasmablasts, B cells, etc.) using methods well known by one of skill in the art, including, for example, Ficoll separation techniques.

The present invention provides an efficient and effective means for identifying rare plasmablasts (and rare B cells) (and their corresponding monoclonal antibodies) having specificity (*e.g.,* able to recognize or bind) to more than one antigen or to different variants, isolates, subtypes, or homologs of the same antigen. In one aspect, the present invention provides methods for identifying an antigen-specific plasmablast which produces an antibody that is able to recognize or bind to more than one antigen or to different variants, isolates, subtypes, or homologs of the same antigen. For example, the present invention shows, by way of example, the use of the present methods to identify rare human monoclonal antibodies directed against hemagglutinin, including monoclonal antibodies that, individually, recognize influenza A virus Group 1 and Group2 hemagglutinin subtypes.

Accordingly, the present methods are useful for identifying an antibody (such as a monoclonal antibody, in particular a human monoclonal antibody) which recognizes, binds, or has specificity to one or more different variants, homologs, subtypes, or isotypes of the same antigen (*e.g.,* a protein, polypeptide, etc.), such as, for example, an antibody which recognizes, binds, or has specificity to the same or homologous antigen from more than one species, to the same or homologous antigen from different species, to the same or homologous antigen common to more than one species (*e.g.,* such as an antibody which recognizes, binds, or has specificity to the same or homologous antigen from a human and a mouse). Such antibodies are particularly useful by virtue of their ability to recognize and/or bind to the same antigen or homologous antigen from different species, subtypes, isolates (*e.g.,* viral isolates), strains, etc. In some embodiments, the present methods are useful for identifying a cross-reactive antibody (*e.g.,* an antibody that binds or is able to recognize the non-human animal antigen as well as the human antigen).

Antibodies obtained by the present methods are useful in various research, medical, therapeutic (e.g., passive immunotherapy), and diagnostic applications.

### Recombinant Methods and Compositions

Antibodies identified using methods of the present invention may be produced using recombinant methods and compositions, *e.g.,* as described in U.S. Patent No. 4,816,567. For example, isolated nucleic acid molecules encoding an antibody identified according to the present invention can be isolated. Such nucleic acid may encode an amino acid sequence comprising the VL and/or an amino acid sequence comprising the VH of the antibody (*e.g.,* the light and/or heavy chains of the antibody). In a further embodiment, one or more vectors (*e.g.,* expression vectors) comprising such nucleic acid are provided. In a further embodiment, a host cell comprising such nucleic acid is provided. In one such embodiment, a host cell comprises (*e.g.,* has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, *e.g.,* a Chinese Hamster Ovary (CHO) cell or lymphoid cell (*e.g.,* Y0, NS0, Sp20 cell).

For recombinant production of an antibody, nucleic acid encoding an antibody, *e.g.,* an antibody identified using methods described herein, is isolated and inserted into one or more vectors for further cloning and/or expression in a host cell. Such nucleic acid may be readily isolated and sequenced using conventional procedures (*e.g.,* by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, *e.g.,* U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, *e.g.,* US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, *e.g.,* Yazaki and Wu, Methods in Molecular Biology, Vol. 248 (B.K.C. Lo, ed., Humana Press, Totowa, NJ), pp. 255-268 (2003).

### EXAMPLES

The following are examples of methods and compositions of the invention. It is understood that various other embodiments may be practiced, given the general description provided above.

### Example 1. Plasmablast enrichment and expansion

The following experiments were performed to evaluate the effect of PBMC expansion and differentiation following intrasplenic transplantation of human peripheral blood mononuclear cells (PBMCs) into SCID/beige mice.

Leukopacs from normal human donors were obtained from Blood Centers of the Pacific (San Francisco, CA). Peripheral blood mononuclear cells (PBMCs) were isolated from the leukopacs using standard methodologies. Six- to eight-week old female SCID/beige mice were purchased from Charles River Laboratories (Hollister, CA) and housed and maintained at Genentech in accordance with American Association of Laboratory Animal Care guidelines. All experimental studies were conducted under the approval of the Institutional Animal Care and Use Committees of Genentech Lab Animal Research in an AAALACi-accredited facility in accordance with the Guide for the Care and Use of Laboratory Animals and applicable laws and regulations. Leukopacs or blood from healthy human donors were obtained after written informed consent was provided and ethical approval granted from the Western Institutional Review Board.

6-8 week old female SCID/beige mice (Charles River Laboratories, Hollister, CA) were sublethally irradiated with 350 rads using a Cesium-137 source. Polymyxin B (110 mg/L) and neomycin (1.1 g/L) were added to the drinking water for 7 days following irradiation. Four hours after irradiation, the left flank of each mouse was shaved and prepped with Betadine® (Purdue Pharma, Stamford, CT) and 70% alcohol. Surgical procedures were performed under anesthesia using aseptic surgical procedures. A 1-cm skin incision was made just below the costal border of each mouse, followed by an incision of the abdominal wall and the peritoneum. The spleen of each mouse was carefully exposed and injected with 50×10⁶ human PBMCs resuspened in 30 µL PBS. The incisions were closed in the muscular layer and in the skin using 5-O Vicryl® sutures (Ethicon, Somerville, NJ) and surgical staples, respectively. Mice were sacrificed at 8 days post-transplantation, and their spleens harvested.

Single cell suspensions of spleen cells were stained with a cocktail of anti-human mAbs (CD38 PECy7, BD Biosciences, San Jose, CA; and CD19) to distinguish B lineage cells.

Figure 1 shows the results of plasmablast differentiation in SCID mice *in vivo.* PBMCs prior to transplant (indicated as day 0) or splenocytes obtained from SCID mice on day 4, 7 and 8 (following intrasplenic injection of human PBMCs) were stained and sorted with anti-CD 19 and anti-CD38 to distinguish B lineage cells. Upper circles, lower circles, and rectangles represent plasmablasts, activated germinal center-like cells, or naive B cells, respectively. Numbers indicate frequencies of each subset as percent. As shown in Figure 1, human plasmablasts expanded to nearly 40% of the total splenic cell population 8 days post-transplantation of 50 million human PBMCs.

### Example 2. Enrichment of tetanus toxin-specific human plasmablasts

Leukopacs from normal human donors that received DT (diphtheria and tetanus toxoid) vaccine 7 days prior to their blood donation were obtained from Blood Centers of the Pacific (San Francisco, CA). Peripheral blood mononuclear cells (PBMCs) were isolated from the leukopacs using standard methodologies. Six- to eight-week old female SCID/beige mice were purchased from Charles River Laboratories (Hollister, CA) and housed and maintained at Genentech in accordance with American Association of Laboratory Animal Care guidelines. All experimental studies were conducted under the approval of the Institutional Animal Care and Use Committees of Genentech Lab Animal Research in an AAALACi-accredited facility in accordance with the Guide for the Care and Use of Laboratory Animals and applicable laws and regulations. Leukopac or blood from healthy human donors was obtained after written informed consent was provided and ethical approval granted from the Western Institutional Review Board.

*In vivo* antigen-driven plasmablast enrichment and expansion was performed using intraspenic transplantation of PBMCs as follows. Isolated PBMCs obtained as described above were resuspended with tetanus toxoid (TT) (List Biological Laboratories, Inc in Campbell, CA) at 0.1-2 µg for each one million B cells and incubated for 30 minutes at 37°C (PBMC/antigen pre-mix). Following the PBMC/antigen pre-mix, the PBMCs were gently washed to remove excess or unbound antigen prior to intra-splenic injection. In this series of experiments, some PBMCs were not subjected to any antigen pre-mix prior to intra-splenic injection in order to compare the effects of antigen pre-mix on the extent or degree of enrichment of tetanus toxin-specific plasmablasts.

6-8 week old female SCID/beige mice (Charles River Laboratories, Hollister, CA) were sublethally irradiated with 350 rads using a Cesium-137 source. Polymyxin B (110 mg/L) and neomycin (1.1 g/L) were added to the drinking water for 7 days following irradiation. Four hours after irradiation, the left flank of each mouse was shaved and prepped with Betadine® (Purdue Pharma, Stamford, CT) and 70% alcohol. Surgical procedures were performed under anesthesia using aseptic surgical procedures. A 1-cm skin incision was made just below the costal border of each mouse, followed by an incision of the abdominal wall and the peritoneum. The spleen of each mouse was carefully exposed and injected with 50×10⁶ human PBMCs resuspened in 30 µL PBS. The incisions were closed in the muscular layer and in the skin using 5-O Vicryl® sutures (Ethicon, Somerville, NJ) and surgical staples, respectively. For antigen-specific cell sorting experiments, mice were sacrificed at 8 days post-transplantation, and their spleens harvested.

Single cell suspensions of spleen cells were stained with a cocktail of anti-human mAbs (CD38 PECy7, BD Biosciences, San Jose, CA; and IgG Dylight, Jackson ImmunoResearch Laboratories, Inc., West Grove, PA) which define human IgG+ plasmablasts as CD38^{high}IgG+ expression. Tetanus toxoid reactive plasmablasts were identified by staining with rTTc.PE (tetanus toxin C-fragment, List Biological Laboratories, Inc., Campbell, CA) and VS38c-FITC (Dako, Carpinteria, CA), a monoclonal mouse antibody which recognizes human p63, a transmembrane protein found in plasma cells.

Samples were then analyzed in the presence of propidium iodide dead cell exclusion on Aria high-speed cell sorter (BD Biosciences, San Jose, CA) and anti-tetanus toxin-specific plasmablasts were sorted in a single cell manner into 96-well tissue culture plates containing 50 µl RPMI cell cutlute media supplemented with 5% Low IgG fetal bovine serum. (Gibco, Grand Island, NY). Five million live cells were recorded for all analysis profiles. Profiles were analyzed by Flowjo version 9.4.11 software.

Upon intrasplenic transplantation of SCID/beige recipients, human peripheral blood mononuclear cells (PBMC) underwent rapid expansion and B-cell activation to differentiate into human plasmablasts. Specifically, human plasmablasts that were not subjected to TT antigen pre-mix expanded to nearly 40% of the total splenic cell population 8 days post-transplantation of 50 million human PBMCs.

To determine whether this process could be improved to enrich for antigen-specific plasmablasts, PBMCs from tetanus-vaccinated human donors were incubated with *Tetanus toxoid* prior to intra-splenic injection (PBMC/antigen pre-mix) in order to provide a survival signal via antigen binding to the B-cell receptor complex. This technique resulted in a 20-fold enrichment of plasmablasts that produce *Tetanus* toxoid-binding monoclonal antibodies, compared to cells that were not incubated with antigen prior to transplant.

Figure 2A shows a representative profile of human donor anti-TT+ PBMCs at day 7 post vaccination. Figure 2B shows a representative profile of anti-TT+ cells taken from the spleen of SCID mice at day 8 after intra-splenic transplantation. Figure 2C shows quantification of anti-TT+ cells per spleen in individual mice under each stimulation condition, as labeled. Cytokine cocktail contained a mixture of human BAFF (50 µg), and IL-2, IL-6, and IL-21 (each at 50 ng) injected ip to hSCID mice.

As shown in Figure 2, of all the conditions examined, the highest number of anti-TT+ plasmablasts was observed in the PBMC/antigen pre-mix using the methods described herein.

These findings indicated that *in vitro* PBMC/antigen pre-mix using PBMCs obtained from tetanus toxoid-vaccinated donors resulted in enrichment of tetanus toxin antigen-specific plasmablasts within the SCID/beige recipient.

### Example 3. Enrichment of influenza A virus hemagglutinin-specific human plasmablasts

Leukopacs from normal human donors that received the seasonal influenza Fluvirin® vaccine (Novartis Lot #111796P1) 7 days prior to their blood donation were obtained from Blood Centers of the Pacific (San Francisco, CA). Peripheral blood mononuclear cells (PBMCs) were isolated from the leukopacs using standard methodologies. Six- to eight-week old female SCID/beige mice were purchased from Charles River Laboratories (Hollister, CA) and housed and maintained at Genentech in accordance with American Association of Laboratory Animal Care guidelines. All experimental studies were conducted under the approval of the Institutional Animal Care and Use Committees of Genentech Lab Animal Research in an AAALACi-accredited facility in accordance with the Guide for the Care and Use of Laboratory Animals and applicable laws and regulations. Leukopac or blood from healthy human donors was obtained after written informed consent was provided and ethical approval granted from the Western Institutional Review Board.

*In vivo* antigen-driven plasmablast enrichment and expansion was performed using intraspenic transplantation of PBMCs as follows. Isolated PBMCs were resuspended with hemagglutinin antigens (0.1-2 µg for each one million B cells) and incubated for 30 minutes at 37°C (PBMC/antigen pre-mix). Following this incubation, the PBMCs were washed to remove unbound antigens. To enrich for plasmablasts that produced cross-reactive hemagglutinin antibodies, the hemagglutinin antigen variants used for PBMC/antigen pre-mix and single cell sorting were specifically chosen to differ from the hemagglutinin antigen variants contained within the influenza Fluvirin® vaccine. Hemagglutinin antigens used in this study, therefore, included H1 hemagglutinin from influenza A virus isolate A/NWS/1933 (a Group1 influenza A virus hemagglutinin), H3 hemagglutinin from influenza A virus isolate A/Hong Kong/8/1968 (a Group2 influenza A virus hemagglutinin), and H7 hemagglutinin from influenza A virus isolate A/Netherlands/219/2003 (a Group2 influenza A virus hemagglutinin). The hemagglutinin antigens were produced at Genentech using standard molecular biology techniques.

6-8 week old female SCID/beige mice (Charles River Laboratories, Hollister, CA) were sublethally irradiated with 350 rads using a Cesium-137 source. Polymyxin B (110 mg/L) and neomycin (1.1 g/L) were added to the drinking water for 7 days following irradiation. Four hours after irradiation, the left flank of each mouse was shaved and prepped with Betadine® (Purdue Pharma, Stamford, CT) and 70% alcohol. Surgical procedures were performed under anesthesia using aseptic surgical procedures. A 1-cm skin incision was made just below the costal border of each mouse, followed by an incision of the abdominal wall and the peritoneum. The spleen of each mouse was carefully exposed and injected with 50×10⁶ human PBMCs resuspened in 30 µL PBS. The incisions were closed in the muscular layer and in the skin using 5-O Vicryl® sutures (Ethicon, Somerville, NJ) and surgical staples, respectively. For antigen-specific cell sorting experiments, mice were sacrificed at 8 days post-transplantation, and their spleens harvested.

Single cell suspensions of spleen cells obtained from the mice were stained with a cocktail of anti-human monoclonal antibodies CD38 PECy7 (BD Biosciences, San Jose, CA) and IgG Dylight (Jackson ImmunoResearch Laboratories, Inc., West Grove, PA) which define human IgG+ plasmablasts as CD38^{high}/IgG+ expression. To identify hemagglutinin cross-reactive plasmablasts within the suspension of isolated spleen cells, the cells were stained with hemagglutinin H1 from influenza virus A isolate A/NWS/1933 and hemagglutinin H3 from influenza virus A isolate A/Hong Kong/8/1968, which were previously conjugated with FITC or PE, respectively, using Lightning-Link® labeling kits (Innova Biosciences, Cambridge, UK).

Figure 3A shows representative FACS analysis of anti-hemagglutinin-positive (H3 and HI) plasmablasts from day 7 post-vaccinated PBMCs prior to SCID/beige mice enrichment (*i.e.,* prior to PBMC/antigen pre-mix). Figure 3B shows representative FACS data analysis of hemagglutinin-positive (H3 and HI) plasmablasts from day 8 post-transplant after SCID/beige mice enrichment, comparing no pre-mix and antigen pre-mix in the upper and lower panels, respectively. As shown in Figures 3A and 3B, PBMC/antigen pre-mix prior to intrasplenic implantation resulted in higher frequency of H3⁺/H1⁺ anti-hemagglutinin reactive plasmablasts.

Table 1 below shows a comparison of anti-H1⁺/anti-H3⁺ plasmablast frequencies before and after SCID enrichment as described herein. As shown in Table 1, the frequency of anti-H1⁺/anti-H3⁺ plasmablasts was greatly increased using the SCID/beige mouse enrichment methods of the present invention compared to that observed without SCID/beige mouse enrichment.

**Table 1**

| Condition | Anti-H1⁺/Anti-H3⁺ Plasmablast Frequency (%) |
|---|---|
| Vaccinated PBMC | 0.00028±0.00008 |
| hSCID + Antigen Premix | 0.011±0.007 |

Samples were then analyzed in the presence of propidium iodide dead cell exclusion on Aria high-speed cell sorter (BD Biosciences, San Jose, CA) and anti-hemagglutinin-specific plasmablasts were sorted in a single cell manner into 96-well tissue culture plates containing 50 µl RPMI cell cutlute media supplemented with 5% Low IgG fetal bovine serum. (Gibco, Grand Island, NY). Five million live cells were recorded for all analysis profiles. Profiles were analyzed by Flowjo version 9.4.11 software.

Figure 4 shows analysis of splenocytes obtained from day-8 post-transplant from individual SCID/beige mice showing stochastic response, comparing no pre-mix (circles) and antigen-premix (squares). Data is presented as percent anti-H1⁺/CD38^{high} plasmablasts. The rectangle indicates mice that presented anti-H1⁺ plasmablasts.

These results showed that broad hemagglutinin cross-reactive plasmablasts were detected if influenza A virus Group1 *(e.g.,* HI) and Group2 *(e.g.,* H3, H7) hemagglutinin antigens were incubated with PBMCs prior to intrasplenic implantation. These results further indicated that *in vitro* stimulation of hemagglutinin antigen-primed PBMCs from influenza-vaccinated donors promoted hemagglutinin antigen-specific enrichment of plasmablasts within the SCID/beige mouse recipients.

### Example 4. IgG cloning from single plasmablasts

Hemagglutinin H1 and H3 cross-reactive human plasmablasts (described above) were single-cell sorted, resulting in approximately 950 plasmablasts. Single plasmablasts were sorted directly into U-bottom 96-well micro-well plates containing 50 µl RPMI containing 5% Low IgG fetal bovine serum. The plates were centrifuged for 5 minutes at 600 x g (Beckman Coulter, Brea, CA) and the media was carefully removed by aspiration. The cells were resuspended and washed twice in 90 µl of PBS following the same procedure.

To generate cDNA encoding the variable heavy chains and light chains, each cell was resuspended in 6 µl of Reverse Transcriptase (RT) reaction mixture containing 2 units RNaseout (Invitrogen, Grand Island, NY), 0.5 mM 4dNTP (Perkin Elmer, Waltham, MA), 1.5 mM MgCl₂, 37.5 mM KCl, 10 mM DTT (dithiothreitol), 0.25% Nonidet P40 (US Biological, Marblehead, MA), 0.1 mg/ml bovine serum albumin (Sigma-Aldrich), 25 mM Tris pH 8.3, 0.25 pmol of IgG₁₋₄ constant, kappa chain constant, and lambda chain constant region specific oligonucleotides (shown below) and 40 U Superscript III (Invitrogen, Grand Island, NY).

| | |
|---|---|
| IgG₁₋₄ constant: | GAAGTAGTCCTTGACCAGGCAG (SEQ ID NO: 1) |
| Kappa constant: | CTCAGCGTCAGGGTGYTGCTGAG (SEQ ID NO: 2) |
| Lambda constant: | GGGTKTGGTSGTCTCCAC (SEQ ID NO: 3) |

The reaction was incubated for 3 x 30-minute intervals at 45°C, 50°C, and 55° C each. Following the incubation, the reaction mixture was diluted to 15 µl with TE buffer (10 mm Tris HCl, 1 mM EDTA). Initial polymerase chain reactions (PCR) were performed to amplify IgG heavy chains, kappa chains, and lambda chains using 2 µl of the diluted RT cocktail from above and Advantage-GC 2 Polymerase Mix (Clontech, Mountain View, CA), following protocols provided by the manufacturers. The PCR amplifications were performed using degenerate oligonucleotides based on variable heavy chain and light chain germline and constant region sequences shown below.

| | | |
|---|---|---|
| IGVH1a | CAGGTGCAGCTGGTGCAGTCTGGGGC | (SEQ ID NO: 4) |
| IGVHlb | CAGGTCCAGCTGGTGCAGTCTGGGGC | (SEQ ID NO: 5) |
| IGVH2 | CAGGTCACCTTGAAGGAGTCTGGTCC | (SEQ ID NO: 6) |
| IGVH3 | GAGGTGCAGCTGGTGGAGTCTGGGGG | (SEQ ID NO: 7) |
| IGVH4 | CAGGTGCAGCTGCAGGAGTCGGGCCC | (SEQ ID NO: 8) |
| IGVH5 | GAGGTGCAGCTGGTGCAGTCTGG | (SEQ ID NO: 9) |
| IGVH6 | CAGGTACAGCTGCAGCAGTCAGGTCC | (SEQ ID NO: 10) |
| IGVH7 | CAGGTGCAGCTGGTGCAATCTGG | (SEQ ID NO: 11) |
| IGKV1 | GHCATCCRGWTGACCCAGTCTC | (SEQ ID NO: 12) |
| IGKV2 | GATRTTGTGATGACYCAGWCTC | (SEQ ID NO: 13) |
| IGKV3 | GAAATWGTRWTGACRCAGTCTC | (SEQ ID NO: 14) |
| IGKV4 | GACATCGTGATGACCCAGTCTCC | (SEQ ID NO: 15) |
| IGKV5 | GAAACGACACTCACGCAGTCTC | (SEQ ID NO: 16) |
| IGKV6 | GAWRTTGTGMTGACWCAGTCTC | (SEQ ID NO: 17) |
| IGLV1 | CAGTCTGTGYTGACKCAGCCRCCCTC | (SEQ ID NO: 18) |
| IGLV2 | CAGTCTGCCCTGACTCAGCCT | (SEQ ID NO: 19) |
| IGLV3 | TCCTATGAGCTGACWCAGSHVCCCKC | (SEQ ID NO: 20) |
| IGLV4 | CAGCCTGTGCTGACTCARTCVCCCTC | (SEQ ID NO: 21) |
| IGLV5 | CAGCCTGTGCTGACTCAGCCAACTTC | (SEQ ID NO: 22) |
| IGLV6 | AATTTTATGCTGACTCAGCCCCAC | (SEQ ID NO: 23) |
| IGLV7 | CAGGCTGTGGTGACTCAGGAGCCC | (SEQ ID NO: 24) |
| IGLV8 | CAGACTGTGGTGACCCAGGAGCC | (SEQ ID NO: 25) |
| IGLV9 | CAGCCTGTGCTGACTCAGCCACC | (SEQ ID NO: 26) |
| HC301.5constant | GCAGCCCAGGGCSGCTGTGC | (SEQ ID NO: 27) |
| Kappa102constant | GCACACAACAGAGGCAGTTCCAG | (SEQ ID NO: 28) |
| Lambda202constant | CTTGRAGCTCCTCAGAGGAG | (SEQ ID NO: 29) |

Heavy chain and light chain PCR amplification reactions were each divided into two reactions as follows: heavy chain families VH.1,2,3 (primers IGVH1a, IGVH1b, IGVH2, IGVH3) and VH.4,5,6,7 (primers IGVH4, IGVH5, IGVH6, and IGVH7); kappa chain families VK. 1,2,3 (primers IGKV1, IGKV2, and IGKV3) and VK.4,5,6 (primers IGVK4, IGVK5, and IGVK6); and lambda chain families VL. 1,2,3,4,5 (IGLV1, IGLV2, IGLV3, IGLV4, and IGLV5) and VL.6,7,8,9 (primers IGLV6, IGLV7, IGLV8, and IGLV9). A touchdown PCR amplification protocol was used for temperature cycling.

Following the reaction, PCR amplification products were treated with Exonuclease1 (Exo) and Shrimp Alkaline Phosphatase (SAP) to remove excess nucleotides and primers from each of the PCR amplification reactions (U.S. Biologicals, Marblehead, MA). Initial PCR amplification products were directly sequenced to determine the variable sequences of both the heavy chains and light chains using Sanger sequencing. Second nested PCR amplifications were performed using germline-matched heavy chain and light chain variable oligonucleotides in order to insert a mammalian signal and constant region cloning sequences using the following oligonucleotide primers.
sVH1a:
   CCACCATGGGATGGTCATGTATCATCCTTTTTCTAGTAGCAACTGCAACTGGAGTACATTCACAGG (SEQ ID NO: 30)
sVH2: 
CCACCATGGGATGGTCATGTATCATCCTTTTTCTAGTAGCAACTGCAACTGGAGTACATTCACAGATC
ACCT (SEQ ID NO: 31)
sVH3vv:
   CCACCATGGGATGGTCATGTATCATCCTTTTTCTAGTAGCAACTGCAACTGGAGTACATTCACAG (SEQ ID NO: 32)
sVH3gl:
   CCACCATGGGATGGTCATGTATCATCCTTTTTCTAGTAGCAACTGCAACTGGAGTACATTCAGAGG (SEQ ID NO: 33)
sVH4: 
CCACCATGGGATGGTCATGTATCATCCTTTTTCTAGTAGCAACTGCAACTGGAGTACATTCACAGGT
GCAGCTGCAGG (SEQ ID NO: 34)
sVH5: 
CCACCATGGGATGGTCATGTATCATCCTTTTTCTAGTAGCAACTGCAACTGGAGTACATTCAGAGGT
GCA (SEQ ID NO: 35)
sVH6: 
CCACCATGGGATGGTCATGTATCATCCTTTTTCTAGTAGCAACTGCAACTGGAGTACATTCACAGGTA
CAGC (SEQ ID NO: 36)
sVH7: 
CCACCATGGGATGGTCATGTATCATCCTTTTTCTAGTAGCAACTGCAACTGGAGTACATTCACAGGT
GCA (SEQ ID NO: 37)
sVK1: 
CCACCATGGGATGGTCATGTATCATCCTTTTTCTAGTAGCAACTGCAACTGGAGTACATTCAGACATC
CAGATGACCCAGTCTCCATCCTCCCTG (SEQ ID NO: 38)
sVK2: 
CCACCATGGGATGGTCATGTATCATCCTTTTTCTAGTAGCAACTGCAACTGGAGTACATTCAGATATT
GTGATGACTCAGTCTCACTCTCCCTGC (SEQ ID NO: 39)
sVK3: 
CCACCATGGGATGGTCATGTATCATCCTTTTTCTAGTAGCAACTGCAACTGGAGTACATTCAGAAATT
GTGTTGACACAGTCTCCAGCCACCCTGTCTTTG (SEQ ID NO: 40)
sVK4: 
CCACCATGGGATGGTCATGTATCATCCTTTTTCTAGTAGCAACTGCAACTGGAGTACATTCAGACATC
GTGATGACCCAGTCTCCAGACTCCCTGGCTGTG (SEQ ID NO: 41)
sVK5: 
CCACCATGGGATGGTCATGTATCATCCTTTTTCTAGTAGCAACTGCAACTGGAGTACATTCAGAAAC
GACACTCACGCAGTCTCCAGC (SEQ ID NO: 42)
sVK6: 
CCACCATGGGATGGTCATGTATCATCCTTTTTCTAGTAGCAACTGCAACTGGAGTACATTCAGAAATT
GTGCTGACTCAGTCTCCAGACTTTCG (SEQ ID NO: 43)
sVL1: 
CCACCATGGGATGGTCATGTATCATCCTTTTTCTAGTAGCAACTGCAACTGGAGTACATTCACAGTCT
GTGYTGACKCAGCCRCCCTC (SEQ ID NO: 44)
sVL2: 
CCACCATGGGATGGTCATGTATCATCCTTTTTCTAGTAGCAACTGCAACTGGAGTACATTCACAGTCT
GCCCTGACTCAGCCT (SEQ ID NO: 45)
sVL3: 
CCACCATGGGATGGTCATGTATCATCCTTTTTCTAGTAGCAACTGCAACTGGAGTACATTCATCCTAT
GAGCTGACWCAGSHVCCCKC (SEQ ID NO: 46)
sVL4: 
CCACCATGGGATGGTCATGTATCATCCTTTTTCTAGTAGCAACTGCAACTGGAGTACATTCACAGCCT
GTGCTGACTCARTCVCCCTC (SEQ ID NO: 47)
sVL5: 
CCACCATGGGATGGTCATGTATCATCCTTTTTCTAGTAGCAACTGCAACTGGAGTACATTCACAGCCT
GTGCTGACTCAGCCAACTTC (SEQ ID NO: 48)
sVL6: 
CCACCATGGGATGGTCATGTATCATCCTTTTTCTAGTAGCAACTGCAACTGGAGTACATTCAAATTTT
ATGCTGACTCAGCCCCAC (SEQ ID NO: 49)
sVL7: 
CCACCATGGGATGGTCATGTATCATCCTTTTTCTAGTAGCAACTGCAACTGGAGTACATTCACAGGCT
GTGGTGACTCAGGAGCCC (SEQ ID NO: 50)
sVL8: 
CCACCATGGGATGGTCATGTATCATCCTTTTTCTAGTAGCAACTGCAACTGGAGTACATTCACAGACT
GTGGTGACCCAGGAGCC (SEQ ID NO: 51)
wVL9: 
CCACCATGGGATGGTCATGTATCATCCTTTTTCTAGTAGCAACTGCAACTGGAGTACATTCACAGCCT
GTGCTGACTCAGCCACC (SEQ ID NO: 52)
Heavy constant: GCCAGGGGGAAGACCGATG (SEQ ID NO: 53)
Kappa constant:
   CTGGGATAGAAGTTATTCAGCAGGCACACAACAGAAGCAGTTCCAGATTTCAACTGCTC (SEQ ID NO: 54)
Lambda constant: CTTGRAGCTCCTCAGAGGAG (SEQ ID NO: 29)

PCR amplification reactions were set up using PrimeStar HS DNA Polymerase with GC (Takara Bio, Shiga, Japan) according to the manufacturer's recommendation. Following the PCR amplification reactions, the amplification products were treated with Exo/SAP as described above. Heavy variable chain and light variable chain encoding PCR amplification products were inserted into a mammalian expression vector using restriction endonuclease free procedures. 20 µl of the PCR amplification products were annealed onto single stranded DNA human templates for IgG₁, kappa, and lambda chain using the Kunkel mutagenesis protocol. (See Kunkel (1985) PNAS 82:488-492.) Correctly inserted constructs were confirmed by DNA sequencing. Plasmids containing nucleic acids encoding heavy chains and light chains were co-transfected into 293T human embryonic kidney cells using Fugene transfection reagent (Roche Diagnostic, Indianapolis, IN) for transient expression, and analyzed for expression and binding as described below in Example 5.

### Example 5. Hemagglutinin ELISA screening assay

The ability of each monoclonal anti-hemagglutinin antibody obtained using the methods of the present invention to bind various hemagglutinin subtypes was examined by ELISA as follows. Various hemagglutinin-expressing plasmids were transfected into 293T cells as described above. These included hemagglutinin H1 from H1N1/South Carolina/1918, hemagglutinin H3 from H3N2/Perth/2009, hemagglutinin H5 from H5N1/Viet/2004, and hemagglutinin H7 from H7N7/Netherlands/2003 influenza A viruses. After two days, cells were lysed in 50 mM Tris, pH 8, 5 mM EDTA, 150 mM NaCl, 1% Triton X-100 plus protease inhibitor cocktail (Roche). Nuclei were cleared by centrifugation and the resulting lysates were stored at -80°C.

For ELISA screening, 384-well plates (Nunc MaxiSorp) were coated with 5 µg/ml Galanthus nivalis lectin (Sigma) in PBS. The plates were washed and then coated with dilutions of the cell lysates containing various expressed hemagglutinins. The plates were washed and incubated with various dilutions of the anti-hemagglutinin antibodies and subsequently with a goat-anti-human-HRP secondary antibody (Jackson). Plates were washed and processed for TMB (3,3',5,5'-tetramethylbenzidine) substrate detection.

Approximately 950 plasmablasts were obtained from single-cell sorting described above in Example 2. Of this, 840 monoclonal antibodies were transiently expressed in 293T cells and screened by ELISA for binding to H1, H3, H5, and H7 hemagglutinin subtypes, resulting in 82 monoclonal antibodies that bound influenza A virus Group 1 or Group2 hemagglutinin, and 20 monoclonal antibodies that bound both influenza A virus Group1 and Group2 hemagglutinins.

### Example 6. In vitro influenza A virus neutralization

The ability of the anti-hemagglutinin antibodies identified using methods of the present invention to elicit broad hemagglutinin subtype binding and neutralization of a panel of influenza A Group 1 and Group2 virus isolates *in vitro* was examined as follows.

MDCK cells were grown in DMEM media supplemented with 10% FBS as a single 25% confluent monolayer in 96-well black with clear bottom imaging plates (Costar 3904). Each influenza A virus subtype/strain was diluted in influenza media (DMEM + 0.2% BSA, 2 µg/ml TPCK treated Trypsin) to an MOI of 1 and incubated for 1 hour at 37°C with varying concentrations (ranging from 0.02 nM to 1,600 nM) of each antibody. Each antibody/influenza virus mixture was allowed to infect MDCK cells for 16 hours at 37°C in a 5% CO₂ incubator prior to fixation of the cells with cold 100% ethanol. The fixed cells were then stained with Hoechst 33342 (Invitrogen, Cat#

H3570) to visualize cell nuclei and determine total cell number. The cells were also stained with a broadly reactive monoclonal antibody (Millipore Cat# MAB8258) specific for influenza A virus nucleoprotein in order to determine the number of infected cells.

Cells were imaged using the Image Express Micro (Molecular Devices) and data images were analyzed using MetaXpress 3.1 software. The percentage of infected cells was determined and plotted on the Y-axis versus the Log 10 antibody concentration on the X-axis. All neutralization assays were completed in triplicate. Data were fit using a nonlinear regression dose-response curve and are presented in Figure 5 as IC₅₀ values in nM with 95% confidence intervals (95% CI).
The hemagglutinin (HA) subtype of each influenza A virus strain is provided in Figure 5.

*In vitro* neutralization dose-response curves were generated using various concentrations of the monoclonal antibodies described herein against a broad panel of influenza A Group 1 and Group2 virus strains. Figures 6A and 6B show neutralization curves of mAb 39.29 against a panel of influenza A Group 1 and Group2 virus strains, respectively. As shown in Figures 6A and 6B, mAb 39.29 was effective at *in vitro* neutralization of all influenza A virus strains tested. Additionally, Figures 7A and 7B show neutralization curves of mAb 81.39 against a panel of influenza A Group 1 and Group2 virus strains, respectively. As shown in Figures 7A and 7B, mAb 81.39 was effective at the *in vitro* neutralization of all influenza A virus strains tested.

These results showed that anti-hemagglutinin antibodies identified using methods of the present invention exhibited neutralization activity against a variety of influenza A virus strains. Specifically, two anti-hemagglutinin antibodies (mAb 39.29 and mAb 81.39) were effective at neutralizing all influenza A virus strains examined, including neutralization of both Group1 influenza A virus strains (A/CA/7/2009, A/Brisbane/59/2007, A/Solomon/3/2006, A/New Caldonia (Caledonia?)/20/1999, A/PR/8/1934, and A/Japan/305/1957) and Group2 influenza A virus strains (A/Victoria/361/2011, A/Perth/16/2009, A/Brisbane/10/2007, A/Wisconsin/67/2005, A/Victoria/3/1975, A/Port Chalmers/1/1973, A/HK/8/1968, and A/Aichi/2/1968).

Taken together, these results showed that using the antigen-specific plasmablast enrichment methodology of the present invention resulted in the identification of monoclonal antibodies having specificity to more than one antigen *(i.e.,* having specificity to various hemagglutinin subtypes). Additionally, these results showed that the plasmablast enrichment methodology described herein resulted in the identification of monoclonal antibodies capable of neutralizing both Group 1 and Group2 influenza A virus strains from only 950 isolated plasmablasts.

### Example 7. In vivo efficacy of mAb 39.29 in mice

The anti-hemagglutinin antibodies obtained using methods of the present invention were further characterized. The *in vivo* efficacy of mAb 39.29 (mAb 39.29 NC v1) to influenza A virus infection in mice was performed as follows. DBA/2J mice (Jackson Lab, Bar Harbor, ME) were infected intranasally with 50 µl of various influenza A virus strains diluted in influenza media (DMEM, 0.2% BSA, 2 µg/mL TPCK-treated trypsin) at the minimum LD₁₀₀ dose. Four different influenza A virus strains exhibiting a range of *in vitro* IC₅₀ values were used in this series of experiments, including: H1N1 A/PR/8/1934 (Genentech; IC₅₀ 2.0 nM), used at 40 PFU per mouse; H3N2 A/Hong Kong/1/1968 (ViraPur, San Diego, CA; IC₅₀ 45.1 nM), used at 3 PFU per mouse; H3N2 A/Port Chalmers/1/1973 (ViraPur, San Diego, CA; IC₅₀ 2.2 nM), used at 1.5x10⁴ PFU per mouse; and H3N2 A/Aichi/2/1968 (ViraPur, San Diego, CA; IC₅₀ 35 nM), used at 2x10² PFU per mouse. Influenza virus infection was allowed to progress for 72 hours prior to the intravenous administration of mAb 39.29.

After 72 hours post influenza virus A infection, various amounts of mAb 39.29 were administered intravenously to the mice at a dose of 900 µg/mouse (approximately 45 mg/kg), 300 µg/mouse (approximately 15 mg/kg), and 100 µg/mouse (approximately 5 mg/kg) in 200 µl PBS. Control treated animals were administered mAb gD5237 (a monoclonal antibody specific for glycoprotein D of herpes simplex virus (HSV)) at the highest tested equivalent dose of mAb 39.29 *(i.e.,* approximately 45 mg/kg). Mice were monitored daily for body conditioning and survival, and also weighed daily, until 21 days after infection. All mAb39.29 doses vs. control in all four influenza A virus strain infections gave a Log-rank test of P<0.01.

Figures 8A, 8B, 8C, and 8D show percent survival (over time, in days) of mice administered various amounts of mAb 39.29 72 hours after infection with influenza A virus A/PR/8/1934, A/Port Chalmers/1/1973, A/Hong Kong/1/1968, and A/Aichi/2/1968, respectively. As shown in Figures 8A, 8B, 8C, and 8D, 100% mortality was observed by day 14 in infected mice administered control antibody. However, infected mice administered monoclonal antibody of the present invention showed increased survival. In particular, 100% survival was observed in mice infected with influenza virus A/Port Chalmers/1/1973 or influenza virus A/Aichi/2/1968 at all doses of 39.29 tested. (See Figures 8B and 8D.)

These results showed that monoclonal antibodies obtained according to the methods of the present invention are effective at treating various influenza A virus subtype infections. Although the foregoing invention has been described in some detail by way of illustration and example for purposes of clarity and understanding, the description and examples should not be construed as limiting the scope of the invention.

## Claims

1. A method for obtaining a plasmablast population enriched for plasmablasts having specificity to an antigen, the method comprising obtaining peripheral blood mononuclear cells (PBMCs) from a donor, contacting *in vitro* the PBMCs with the antigen to obtain a PBMC/antigen pre-mix, transferring the PBMCs into the spleen of an immunocompromised non-human animal, and isolating the plasmablasts from the spleen of the immunocompromised animal, thereby obtaining a plasmablast population enriched for plasmablasts having specificity to an antigen.

2. The method of claim 1, wherein the method further comprises washing the PBMCs to remove excess or unbound antigen from the PBMC/antigen pre-mix prior to transferring the PBMCs into the spleen of the immunocompromised animal.

3. The method of claim 1, wherein the donor is a donor previously exposed to the antigen.

4. The method of claim 1, wherein the method further comprises single-cell sorting of the plasmablasts.

5. The method of claim 1, wherein the method further comprises antigen-specific single-cell sorting of the plasmablasts.

6. The method of claim 1, wherein the method further comprises cloning of immunoglobulin from the plasmablasts.

7. A method for enriching antigen-specific plasmablasts, the method comprising obtaining peripheral blood mononuclear cells (PBMCs) from a donor, contacting *in vitro* the PBMCs with at least one antigen to obtain an PBMC/antigen pre-mix, transferring the PBMCs into the spleen of an immunocompromised non-human animal, and isolating the plasmablasts from the spleen of the immunocompromised animal, thereby enriching antigen-specific plasmablasts.

8. The method of claim 7, wherein the method further comprises washing the PBMCs to remove excess or unbound antigen from the PBMC/antigen pre-mix prior to transferring the PBMCs into the spleen of the immunocompromised animal.

9. The method of claim 7, wherein the donor is a donor previously exposed to the antigen.

10. The method of claim 7, wherein the method further comprises single-cell sorting of the plasmablasts.

11. The method of claim 7, wherein the method further comprises antigen-specific single-cell sorting of the plasmablasts.

12. The method of claim 7, wherein the method further comprises cloning of immunoglobulin from the plasmablasts.

13. The method of claim 7, wherein the method further comprises contacting the PBMCs with two or more different antigens.

14. A method for a identifying a plasmablast capable of producing an antibody having specificity to an antigen, the method comprising obtaining peripheral blood mononuclear cells (PBMCs) from a donor, contacting *in vitro* the PBMCs with at least one antigen to obtain a PBMC/antigen pre-mix, transferring the PBMCs into the spleen of an immunocompromised non-human animal, isolating the plasmablasts from the spleen of the immunocompromised animal, and antigen-specific cell sorting of the isolated plasmablasts, thereby identifying a plasmablast capable of producing an antibody having specificity to an antigen.

15. The method of claim 14, wherein the method further comprises washing the PBMCs to remove excess or unbound antigen from the PBMC/antigen pre-mix prior to transferring the PBMCs into the spleen of the immunocompromised.

16. The method of claim 14, wherein the method further comprises cloning of immunoglobulin from the isolated plasmablast.

17. A method for a identifying a plasmablast capable of producing an antibody having specificity to more than one antigen, the method comprising obtaining peripheral blood mononuclear cells (PBMCs) from a donor, contacting *in vitro* the PBMCs with two or more different antigens to obtain a PBMC/antigen pre-mix, transferring the PBMCs into the spleen of an immunocompromised non-human animal, isolating the plasmablasts from the spleen of the immunocompromised animal, and antigen-specific cell sorting of the isolated plasmablasts, thereby identifying a plasmablast capable of producing an antibody having specificity to more than one antigen.

18. The method of claim 17, wherein the method further comprises washing the PBMCs to remove excess or unbound antigen from the PBMC/antigen pre-mix prior to transferring the PBMCs into the spleen of the immunocompromised.

19. The method of claim 17, wherein the method further comprises cloning of immunoglobulin from the isolated plasmablast.

## Patentansprüche

1. Verfahren zur Gewinnung einer Plasmablasten-Population, die mit Plasmablasten angereichert ist, die Spezifität für ein Antigen aufweisen, wobei das Verfahren das Gewinnen von mononukleären Zellen des peripheren Blutes (PBMC) von einem Spender, das *In-vitro*-Inkontaktbringen der PBMC mit dem Antigen, um eine PBMC/Antigen-Vormischung zu erhalten, das Übertragen der PBMC in die Milz eines immunsupprimierten nicht-menschlichen Tieres und das Isolieren der Plasmablasten aus der Milz des immunsupprimierten Tieres, umfasst, dadurch wird eine Plasmablasten-Population erhalten, die mit Plasmablasten angereichert ist, die Spezifität für ein Antigen aufweisen.

2. Verfahren nach Anspruch 1, wobei das Verfahren ferner das Waschen der PBMC umfasst, um überschüssiges oder ungebundenes Antigen aus der PBMC/Antigen-Vormischung zu entfernen, bevor die PBMC in die Milz des immunsupprimierten Tieres übertragen werden.

3. Verfahren nach Anspruch 1, wobei der Spender ein Spender ist, der zuvor dem Antigen ausgesetzt war.

4. Verfahren nach Anspruch 1, wobei das Verfahren ferner das Einzelzell-Sortieren der Plasmablasten umfasst.

5. Verfahren nach Anspruch 1, wobei das Verfahren ferner das antigenspezifische Einzelzell-Sortieren der Plasmablasten umfasst.

6. Verfahren nach Anspruch 1, wobei das Verfahren ferner das Klonieren von Immunglobulin aus den Plasmablasten umfasst.

7. Verfahren zur Anreicherung von antigenspezifischen Plasmablasten, wobei das Verfahren das Gewinnen von mononukleären Zellen des peripheren Blutes (PBMC) von einem Spender, das *In-vitro*-Inkontaktbringen der PBMC mit mindestens einem Antigen, um eine PBMC/Antigen-Vormischung zu erhalten, das Übertragen der PBMC in die Milz eines immunsupprimierten nicht-menschlichen Tieres und das Isolieren der Plasmablasten aus der Milz des immunsupprimierten Tieres, umfasst, dadurch werden antigenspezifische Plasmablasten angereichert.

8. Verfahren nach Anspruch 7, wobei das Verfahren ferner das Waschen der PBMC umfasst, um überschüssiges oder ungebundenes Antigen aus der PBMC/Antigen-Vormischung zu entfernen, bevor die PBMC in die Milz des immunsupprimierten Tieres übertragen werden.

9. Verfahren nach Anspruch 7, wobei der Spender ein Spender ist, der zuvor dem Antigen ausgesetzt war.

10. Verfahren nach Anspruch 7, wobei das Verfahren ferner das Einzelzell-Sortieren der Plasmablasten umfasst.

11. Verfahren nach Anspruch 7, wobei das Verfahren ferner das antigenspezifische Einzelzell-Sortieren der Plasmablasten umfasst.

12. Verfahren nach Anspruch 7, wobei das Verfahren ferner das Klonieren von Immunglobulin aus den Plasmablasten umfasst.

13. Verfahren nach Anspruch 7, wobei das Verfahren ferner das Inkontaktbringen der PBMC mit zwei oder mehr verschiedenen Antigenen umfasst.

14. Verfahren zur Identifizierung eines Plasmablasten, der fähig ist, einen Antikörper zu produzieren, der eine Spezifität für ein Antigen aufweist, wobei das Verfahren das Gewinnen von mononukleären Zellen des peripheren Blutes (PBMC) von einem Spender, das *In-vitro*-Inkontaktbringen der PBMC mit mindestens einem Antigen, um eine PBMC/Antigen-Vormischung zu erhalten, das Übertragen der PBMC in die Milz eines immunsupprimierten nicht-menschlichen Tieres, das Isolieren der Plasmablasten aus der Milz des immunsupprimierten Tieres und das antigenspezifische Zellsortieren der isolierten Plasmablasten, umfasst, dadurch wird ein Plasmablast identifiziert, der fähig ist, einen Antikörper zu produzieren, der eine Spezifität für ein Antigen aufweist.

15. Verfahren nach Anspruch 14, wobei das Verfahren ferner das Waschen der PBMC umfasst, um überschüssiges oder ungebundenes Antigen aus der PBMC/Antigen-Vormischung zu entfernen, bevor die PBMC in die Milz des Immunsupprimierten übertragen werden.

16. Verfahren nach Anspruch 14, wobei das Verfahren ferner das Klonieren von Immunglobulin aus dem isolierten Plasmablast umfasst.

17. Verfahren zur Identifizierung eines Plasmablasten, der fähig ist, einen Antikörper zu produzieren, der Spezifität für mehr als ein Antigen aufweist, wobei das Verfahren das Gewinnen von mononukleären Zellen des peripheren Blutes (PBMC) von einem Spender, das *In-vitro*-Inkontaktbringen der PBMC mit zwei oder mehr verschiedenen Antigenen, um eine PBMC/Antigen-Vormischung zu erhalten, das Übertragen der PBMC in die Milz eines immunsupprimierten nicht-menschlichen Tieres, das Isolieren der Plasmablasten aus der Milz des immunsupprimierten Tieres und das antigenspezifische Zellsortieren der isolierten Plasmablasten, umfasst, dadurch wird ein Plasmablast identifiziert, der fähig ist, einen Antikörper zu produzieren, der Spezifität für mehr als ein Antigen aufweist.

18. Verfahren nach Anspruch 17, wobei das Verfahren ferner das Waschen der PBMC umfasst, um überschüssiges oder ungebundenes Antigen aus der PBMC/Antigen-Vormischung zu entfernen, bevor die PBMC in die Milz des Immunsupprimierten übertragen werden.

19. Verfahren nach Anspruch 17, wobei das Verfahren ferner das Klonieren von Immunglobulin aus dem isolierten Plasmablast umfasst.

## Revendications

1. Procédé d'obtention d'une population de plasmoblastes enrichie en plasmoblastes présentant une spécificité envers un antigène, le procédé comprenant l'obtention de cellules mononucléaires du sang périphérique (PBMC) à partir d'un donneur, la mise en contact *in vitro* des PBMC avec l'antigène pour obtenir un prémélange PBMC/antigène, le transfert des PBMC dans la rate d'un animal non humain immunocompromis, et l'isolement des plasmoblastes de la rate de l'animal immunocompromis, ce qui permet l'obtention d'une population de plasmoblastes enrichie en plasmoblastes présentant une spécificité envers un antigène.

2. Procédé selon la revendication 1, ledit procédé comprenant en outre le lavage des PBMC pour enlever l'antigène en excès ou non lié du prémélange PBMC/antigène avant le transfert des PBMC dans la rate de l'animal immunocompromis.

3. Procédé selon la revendication 1, dans lequel le donneur est un donneur préalablement exposé à l'antigène.

4. Procédé selon la revendication 1, ledit procédé comprenant en outre le tri unicellulaire des plasmoblastes.

5. Procédé selon la revendication 1, ledit procédé comprenant en outre le tri unicellulaire spécifique d'antigènes des plasmoblastes.

6. Procédé selon la revendication 1, ledit procédé comprenant en outre le clonage de l'immunoglobuline provenant des plasmoblastes.

7. Procédé d'enrichissement de plasmoblastes spécifiques d'antigènes, le procédé comprenant l'obtention de cellules mononucléaires du sang périphérique (PBMC) à partir d'un donneur, la mise en contact *in vitro* des PBMC avec au moins un antigène pour obtenir un prémélange PBMC/antigène, le transfert des PBMC dans la rate d'un animal non humain immunocompromis, et l'isolement des plasmoblastes provenant de la rate de l'animal immunocompromis, ce qui permet l'enrichissement de plasmoblastes spécifiques d'antigènes.

8. Procédé selon la revendication 7, ledit procédé comprenant en outre le lavage des PBMC pour enlever l'antigène en excès ou non lié du prémélange PBMC/antigène avant le transfert des PBMC dans la rate de l'animal immunocompromis.

9. Procédé selon la revendication 7, dans lequel le donneur est un donneur préalablement exposé à l'antigène.

10. Procédé selon la revendication 7, ledit procédé comprenant en outre le tri unicellulaire des plasmoblastes.

11. Procédé selon la revendication 7, ledit procédé comprenant en outre le tri unicellulaire spécifique d'antigènes des plasmoblastes.

12. Procédé selon la revendication 7, ledit procédé comprenant en outre le clonage de l'immunoglobuline provenant des plasmoblastes.

13. Procédé selon la revendication 7, ledit procédé comprenant en outre la mise en contact des PBMC avec deux antigènes différents ou plus.

14. Procédé d'identification d'un plasmoblaste pouvant produire un anticorps présentant une spécificité envers un antigène, le procédé comprenant l'obtention de cellules mononucléaires du sang périphérique (PBMC) à partir d'un donneur, la mise en contact *in vitro* des PBMC avec au moins un antigène pour obtenir un prémélange PBMC/antigène, le transfert des PBMC dans la rate d'un animal non humain immunocompromis, l'isolement des plasmoblastes de la rate de l'animal immunocompromis, et le tri cellulaire spécifique d'antigènes des plasmoblastes isolés, ce qui permet l'identification d'un plasmoblaste pouvant produire un anticorps présentant une spécificité envers un antigène.

15. Procédé selon la revendication 14, ledit procédé comprenant en outre le lavage des PBMC pour enlever l'antigène en excès ou non lié du prémélange PBMC/antigène avant le transfert des PBMC dans la rate de l'animal immunocompromis.

16. Procédé selon la revendication 14, ledit procédé comprenant en outre le clonage de l'immunoglobuline provenant du plasmoblaste isolé.

17. Procédé d'identification d'un plasmoblaste pouvant produire un anticorps présentant une spécificité envers plus d'un antigène, le procédé comprenant l'obtention de cellules mononucléaires du sang périphérique (PBMC) à partir d'un donneur, la mise en contact *in vitro* des PBMC avec deux antigènes différents ou plus pour obtenir un prémélange PBMC/antigène, le transfert des PBMC dans la rate d'un animal non humain immunocompromis, l'isolement des plasmoblastes de la rate de l'animal immunocompromis, et le tri cellulaire spécifique d'antigènes des plasmoblastes isolés, ce qui permet l'identification d'un plasmoblaste pouvant produire un anticorps présentant une spécificité envers plus d'un antigène.

18. Procédé selon la revendication 17, ledit procédé comprenant en outre le lavage des PBMC pour enlever l'antigène en excès ou non lié du prémélange PBMC/antigène avant le transfert des PBMC dans la rate de l'animal immunocompromis.

19. Procédé selon la revendication 17, ledit procédé comprenant en outre le clonage de l'immunoglobuline provenant du plasmoblaste isolé.
